# EUROPEAN PATENT SPECIFICATION

(11) **EP 1 824 556 B1**
(45) Date of publication and mention of the grant of the patent: **19.01.2011**
(21) Application number: 05779831.6
(22) Date of filing: 01.08.2005
(51) Int. Cl.: A61M 39/22, B29C 45/00, F16K 11/08

(54) **A FLUID CONTROL VALVE**
FLÜSSIGKEITSKONTROLLVENTIL
VALVE DE REGULATION DE DEBIT

(30) Priority: 26.11.2004 IT MO20040312
(43) Date of publication of application: 29.08.2007
(73) Proprietor: Gambro Lundia AB, 22010 Lund (SE)
(72) Inventor: COLLI, Guido, I-41100 Modena (IT); RICCO', Cristina, I-41100 Modena (IT)
(74) Representative: Ponzellini, Gianmarco
(86) International application number: PCT/IB2005/002262
(87) International publication number: WO 2006/056827

(56) References cited:
- WO-A-88/06895
- US-A- 4 103 865
- US-A- 4 432 392
- US-A1- 2003 181 850

## Description

### Background of the Invention

The invention relates to a fluid control valve and a peritoneal dialysis set comprising the valve.

Specifically, though not exclusively, the invention can be usefully applied for control of fluid distribution in a peritoneal dialysis treatment.

Patent publication WO 2004/022151 teaches a valve in which the valve body is made of a polycarbonate and the selector is made of polyethylene.

A valve made in this way can be improved in various ways.

Firstly, the efficiency of the liquid seal in the coupling zone between the valve body and the rotating selector is susceptible to improvement.

Secondly, the reliability and the working life, as well as the fluid seal capacity, of the coupling between the control valve and the three flexible fluid transport tubes to each of the three access ports of the valve are susceptible to improvement.

Obtaining the above-cited improvement must be linked to questions of biocompatibility, connected to the medical use of the valve, and also must take account of the fact that the improving characteristics must survive steam sterilisation treatment at high temperature, which the valve is normally subjected to.

The above improvements must be made in the light of the need to contain costs, to simplify the manufacturing process and to ensure product reliability.

US 4432392 **discloses a valve as in the preamble of claim 1.**

### Summary of the Invention

A prime aim of the present invention is to provide a fluid control valve which is improved in all of the above-cited aspects.

A further aim of the invention is to realise a peritoneal dialysis set which includes a valve made according to the invention.

An advantage of the invention is to provide a valve having high seal efficiency between the valve body and the mobile selector.

A further advantage is to guarantee the effectiveness of the seal between the valve body and the selector even after a steam sterilisation treatment.

A further advantage is to make available a valve which is associable to fluid transport tubes by a simple, economical and reliable coupling. The coupling can advantageously be obtained without any use of glue.

A still further advantage is to provide a valve which can be used for medical purposes, such as for example in a peritoneal dialysis set.

A still further advantage is to realise a peritoneal dialysis set which is easy and economical to manufacture and very reliable.

In a specific embodiment of the invention, the valve body is realised in two parts with different characteristics, in which one part defines the internal cavity which receives the operative element of the fluid distribution selector, and the other part defines the two or more access ports destined to be coupled with the fluid transport tubes.

In a specific embodiment of the invention, the valve body is made of a plastic material by co-moulding or over-moulding of the two above-mentioned parts.

In a specific embodiment of the invention, the part of the valve body that defines the internal cavity is made of a semi-crystalline plastic material and the part defining the access ports is realised in an amorphous plastic material.

In a specific embodiment of the invention, the part of the valve body defining the internal cavity has a structure which, in comparison with the part that defines the access ports, is provided with greater stability when subjected to heating.

In a specific embodiment of the invention, the part of the valve body defining the internal cavity is less deformable on heating than the part which defines the access ports.

In a specific embodiment of the invention, the part of the valve body defining the internal cavity has an ability to conserve its characteristics if subjected to heating, especially its geometrical characteristics, which ability is greater with respect to that of the part defining the access ports.

In a specific embodiment of the invention, the valve body is made in two parts having different behaviours when undergoing heating-up, so that the part of the valve body defining the internal cavity maintains an effective fluid seal with the selector, while the part defining the fluid access ports is couplable simply and economically to a tube made of plastic material of a peritoneal dialysis set.

Further characteristics and advantages of the present invention will better emerge from the detailed description that follows of at least one preferred embodiment of the invention, illustrated purely by way of non-limiting example in the accompanying figures of the drawings.

### Brief Description of the Drawings

The description will now be made with reference to the accompanying figures of the drawings, which are provided by way of illustration and are therefore not limiting, in which:
- figure 1 is a perspective view from below of a valve body of a fluid control valve according to the present invention;
- figure 2 is a perspective view from above of the valve body of figure 1;

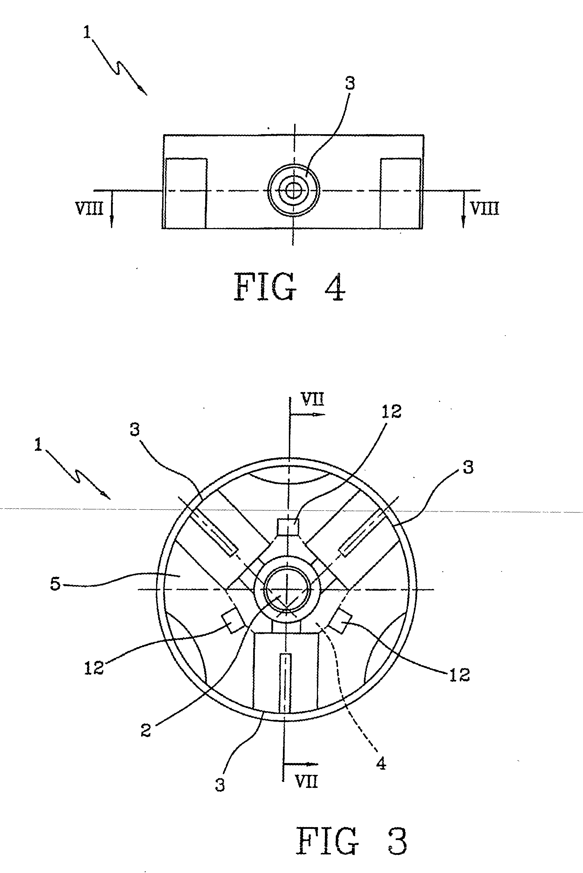

- figure 3 is a plan view from above of the valve body of figure 1;
- figure 4 is a view from below of figure 3;

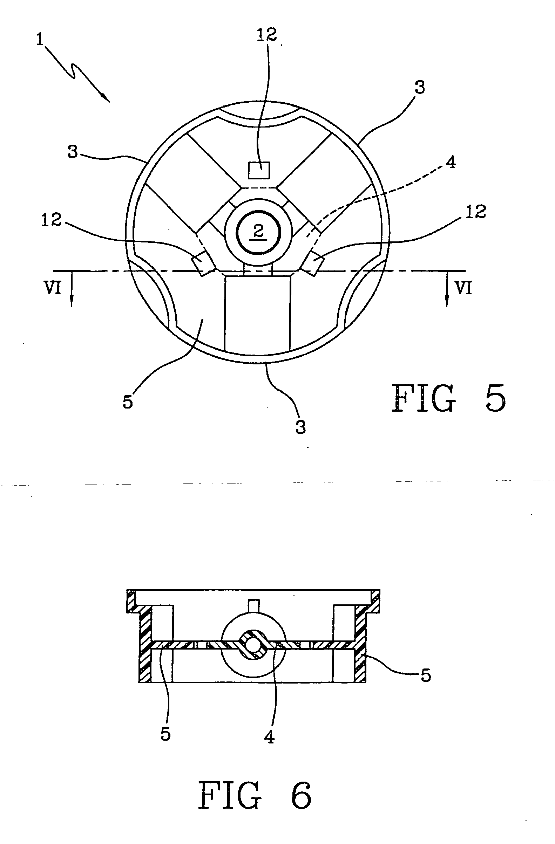

- figure 5 is a view from below of figure 4;
- figure 6 is a section according to line VI-VI of figure 5;

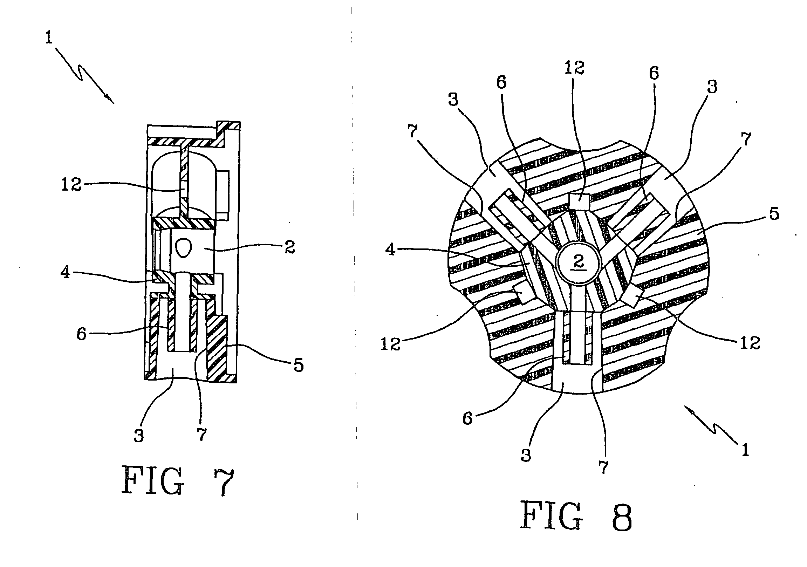

- figure 7 is a section according to line VII-VII of figure 3;
- figure 8 is a section according to line VIII-VIII of figure 4;

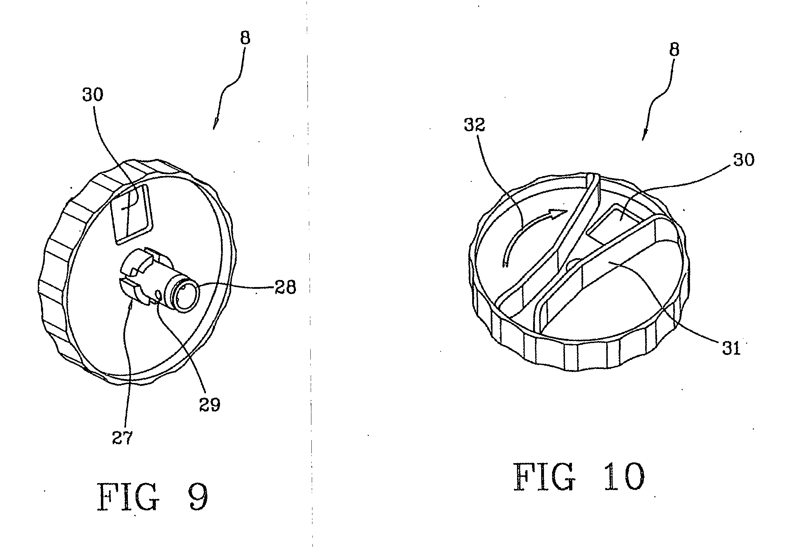

- figure 9 is a perspective view from below of a selector destined for coupling with the valve body of the preceding figures, for realising the valve of the present invention;
- figure 10 is a perspective view from above of the selector of figure 9;
- figure 11 is a lateral view of the selector of figure 9;
- figure 12 is a section according to line XII-XII of figure 11;
- figure 13 is a plan view from above of an auxiliary element destined for coupling with the valve body of figure 1;
- figure 14 is an enlarged detail of figure 13, comprising means for positioning associable to the selector of figure 9;
- figure 15 is a lateral view, partially sectioned, of the auxiliary element of figure 13;

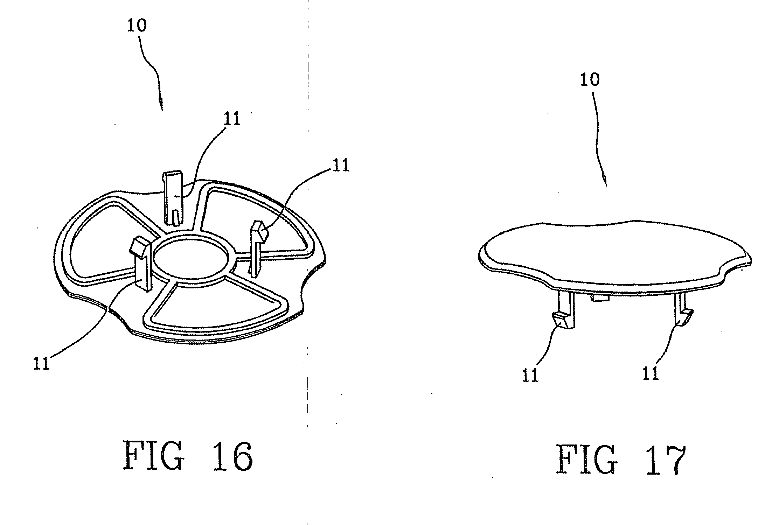

- figures 16 and 17 are two different perspective views of a cover which is couplable to the valve body of figure 1;
- figure 18 is a schematic view of a peritoneal dialysis set.

### Detailed Description

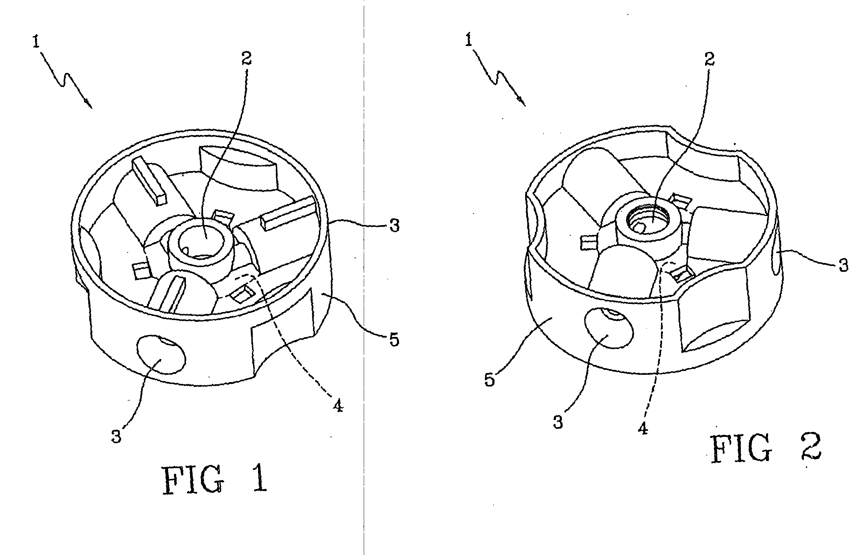

With reference to figure 1 of the drawings, 1 denotes in its entirety a valve body of a fluid control valve for fluid distribution in a peritoneal dialysis set.

The valve body 1 has a peripheral lateral surface, substantially cylindrical, which exhibits three angularly distanced recesses and which has a height which is smaller than a diameter thereof.

The valve body 1 has a cylindrical internal cavity 2, arranged at the centre of the valve body 1. The internal cavity 2 is open at at opposite ends, upper and lower.

The valve body 1 has a plurality of fluid access ports 3, each of which communicates with the internal cavity 2 through a respective radial conduit afforded internally of the valve body 1. There are three access ports 3, arranged radially with respect to the central cavity 2 and angularly distanced one from another. Each access port 3 has a longitudinal axis which meets a central zone of the valve body 1.

The valve body 1 comprises a central first part 4 and a peripheral second part 5 connected to one another solidly and unremovably. The central first part 4 and the peripheral second part 5 are both made of a plastic material.

The first part 4 at least partially defines the internal cavity 2. while the second part at least partially defines the access ports 3.

The second part 5 is in contact with the first part 4. The contact is along an extended zone at least partly circumferential. The border surface between the central first part 4 and the peripheral second part 5 surrounds the internal cavity 2. This border surface is arranged internally with respect to the access ports 3. Also, the border surface, which divides the two parts 4 and 5, extends from one side to another of the valve body 1 for the entire breadth thereof.

The first part 4 forms a central nucleus which is surrounded by the second part 5. In the preferred embodiment the central nucleus is contactingly circumferentially embraced by the second part 5.

More specifically, the second part 5 is a product that is over-moulded on the first part 4.

The first part 4 is made of a first material, which in the preferred embodiment is a crystalline or semi-crystalline plastic material, such as for example a polybutylene terephthalate (PBT). The first material is quite highly crystalline and is thus provided with high resistance to temperature.

The second part 5 is made of a second material, different from the first in at least one chemical-physical property. In the preferred embodiment the chemical-physical property is crystallinity, which is greater in the first material (of the central first part 4) than in the second material (of the peripheral second part 5). More specifically, the second material is an amorphous plastic material and is different from the first material. The second material is a plastic material, which can be amorphous or even not amorphous, but which in any case is susceptible to being coupled without need for glue to a plastic tube, for example a tube made of soft PVC, by contact of the surfaces and heating up to a determined temperature (for example the temperature of steam sterilisation of a medical article which might be a peritoneal dialysis set). In the preferred embodiment described herein, the second material of the second part 5 comprises a polycarbonate.

The central first part 4 defines a plurality of conduits, each of which connects a respective fluid access port 3 with the internal cavity 2.

Each access port 3 comprises a tubular body 6 and a wall 7 which surrounds the tubular body 6. Each tubular body 6 has a longitudinal axis having a component which is directed prevalently radially of the internal cavity 2.

An annular space is defined between the tubular body 6 and the wall 7. The annular space has an annular opening, situated at a first end, for enabling insertion and solid coupling of an end of a flexible fluid transport tube which is part of the dialysis set. In a coupling configuration, the tubular body 6 is inserted at least partially internally of the end of the flexible tube. The annular space is closed at a second end thereof, opposite to the first end, by a bottom wall formed by the material of the valve body. The wall 7 is coaxial and external of the tubular body 6. With reference to a longitudinal axis of the tubular body 6, the wall 7 axially continues beyond the annular end opening. The wall 7 is longer than the tubular body 6. The wall 7 exhibits a peripheral end which defines a circular opening, axially distant from the annular opening. The above-described configuration of the access ports 3 contributes to facilitating the coupling operations of the flexible fluid transport tubes to the ports, apart from also facilitating the stability of the coupling.

The fluid valve control comprises a selector 8 coupled to the valve body 1 and mobile with respect thereto, among a plurality of operative positions, for preventing and/or permitting fluid communication among the access ports 3. In the preferred embodiment the selector 8 is rotary. The selector 8 is realised in plastic material, for example polyethylene.

The first part 4 of the valve body has an internal surface which is axial-symmetric, which in the preferred embodiment is essentially cylindrical and defines the internal cavity 2. The selector 8 has a part which is arranged internally of the cavity 2 and is provided with an external surface, counter-shaped with respect to the internal surface and rotatably coupled there-with. The selector 8 also has a part, external of the cavity 2, which is provided with a manual grip to enable the user easily to rotate the selector.

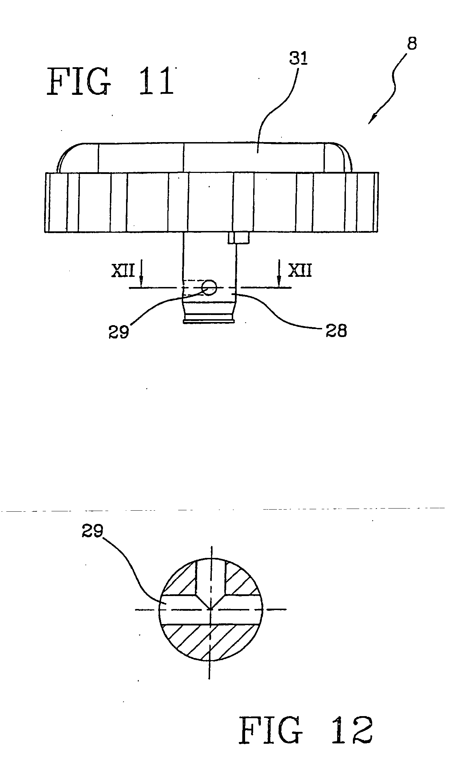

The part of the selector 8 which is internal of the internal cavity 2 functions as a fluid distributor. It comprises a T-shaped channel (figure 12), provided with three end openings, which selectively places in communication the three fluid access ports 3 of the valve body.

The selector 8 can assume at least four operative positions, each of which corresponds to a stage of a peritoneal dialysis treatment: start of treatment, drainage stage of the used liquid from the peritoneal cavity of the patient, dialysis set washing stage, filling stage of the peritoneal cavity with fresh liquid. A fifth position, for example, can be included, for an end treatment stage. The use process of the selector is more specifically described in WO 2004/022151.

The valve is provided with a layer of water-repellent material, interpositioned between the selector 8 and the valve body 1. The water-repellent material, which can form a continuous or discontinuous layer, fills the cavities or hollow spaces situated between the conjoined surfaces of the selector 8 and the valve body 1, which cavities or hollows spaces are due to the roughness of the conjoined surfaces. The water-repellent layer is in contact with both the valve body 1 and the selector 8. More specifically, the water-repellent layer covers a surface of the valve body 1 which delimits the internal cavity 2 and which is coupled to the selector 8. The water-repellent layer is distributed on and adheres to the axial-symmetrical surface which contains the part of the selector 8 that distributes the fluids.

The water-repellent layer can cover a surface of the selector 8 which is coupled with the valve body 1, or there can be two water-repellent layers, one associated to the valve body 1 and the other to the selector 8.

The material that forms the water-repellent layer comprises an oily/greasy substance. In the preferred embodiment the oily/greasy layer comprises a silicone oil.

The material composing the water-repellent layer has a viscosity which is comprised between 500 mm²/s and 2000 mm²/s. An oily material can be used having a viscosity of between 800 mm²/s and 1250 mm²/s. In the preferred embodiment the viscosity of the material used is about 1000 mm²/s. The material forming the water-repellent layer is biocompatible and exhibits a high resistance to high temperatures: in particular the layer conserves its water-repelling characteristics as well as its adherence to the plastic material of the valve surface on which it is distributed, even after a steam sterilisation process.

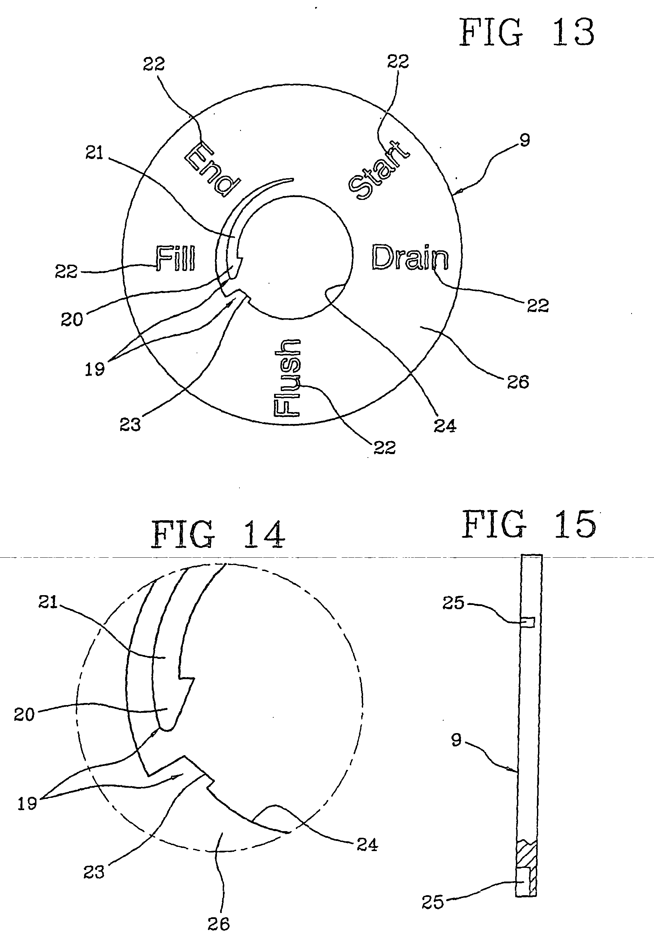

The valve further comprises an auxiliary body 9, illustrated in figures 13 and 14, associated to the valve body 1 and provided with means for positioning the selector 8. The structure and the functioning of the auxiliary body 9 are described in WO 2004/022151.

The valve further comprises a cover 10, made of a plastic material, for protecting the lower side of the valve body 1, i.e. the side opposite the side which, after valve assembly, exhibits the manual grip of the selector 8

The cover 10 is provided with removable means for fixing, which comprise a plurality of flexible tongues 11 emerging from the lower side of the cover 10. Each tongue 11 exhibits an end which is solidly constrained to the cover 10 and a free opposite end which bears a fastening tooth destined, during the assembly stage, to be jointed in a corresponding hole 12 afforded in a bottom wall of the lower side of the valve body 1.

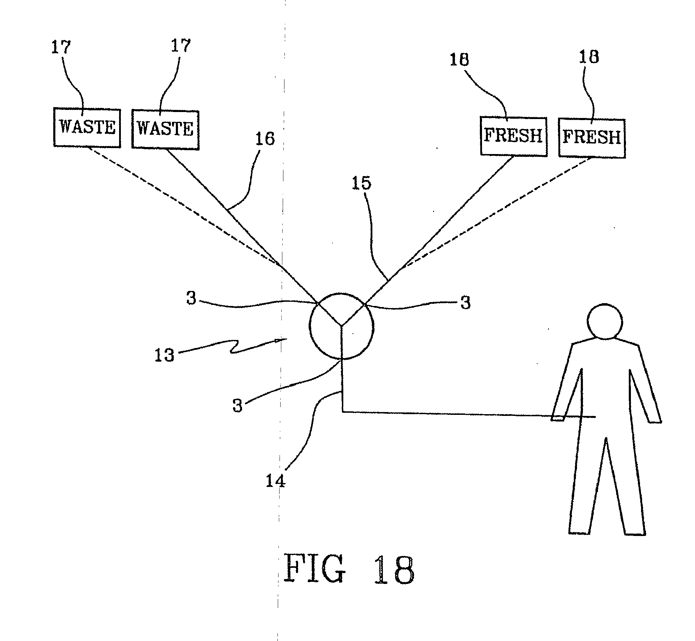

Figure 18 schematically illustrates a peritoneal dialysis set comprising a valve 13 obtained by assembling the components described herein above (valve body 1, selector 8, auxiliary body 9 and cover 10), a plurality of fluid transport tubes 14, 15 and 16, each of which is coupled to a respective access port 3 of the valve 13, one or more containers 17 of the used dialysis liquid, one or more containers 18 of the fresh dialysis liquid. The transport tubes comprise a connecting tube 14 for connection to the patient's peritoneal cavity, an infeed tube 15 of the fresh dialysis fluid connected to the container 18, and a drainage tube 16 of a used dialysis fluid connected to the container 17.

The structure and use of the peritoneal dialysis set of figure 18 is described in WO 2004/022151.

The process for producing the fluid control valve described herein above comprises stages of:
- plastic material injection moulding of a first part of the valve body, comprising the internal cavity and the various relatively short conduits which terminate in the internal cavity;
- overmoulding, by plastic material injection, around the first part, of the second part of the valve body, comprising the various fluid access ports in such a way that each of these ports constitutes a prolongation of the short conduits afforded in the first part of the valve body;
- coupling the auxiliary body to the valve body;
- housing the selector in the internal cavity by insertion thereof through the upper opening of the cavity;
- fixing the cover to the lower side of the valve body.

The above process may comprise a further stage of distribution of the layer of water-repellent material on the surface delimiting the internal cavity, and/or on the external surface of the selector.

A further process for production of a fluid control valve comprises the stages of:
- moulding by plastic material injection a valve body comprising an internal cavity and a plurality of fluid access ports communicating with the cavity;
- providing a selector;
- distributing at least a layer of water-repellent material on at least one from a surface of the valve body which delimits the internal cavity and a surface of the selector;
- housing the selector in the cavity of the valve body so that the water-repellent layer is interpositioned, with a fluid seal function, between the valve body and the selector.

The valve of the present invention has a very reliable seal between the valve body and the selector. The efficiency of the seal is maintained even after a steam sterilisation treatment. Furthermore, the valve can be easily and economically coupled to fluid transport tubes, for example made of PVC, by a coupling process which does not require the use of glue, such as for example simply by heating. The connections between the transport tubes and the access ports of the valve are extremely strong and stable, even in the absence of glue.

### Legend

- 1: Valve body
- 2: Central internal cavity of the valve body
- 3: Fluid access ports of the valve body
- 4: Central first part of the valve body
- 5: Peripheral second part of the valve body
- 6: Tubular body of the access ports
- 7: Wall of the valve body surrounding the tubular body of the access ports
- 8: Fluid distribution selector
- 9: Auxiliary body
- 10: Cover of the valve body
- 11: Flexible fastening tongues of the cover
- 12: Holes on the valve body for fastening the cover
- 13: Valve in its entirety
- 14: Connection tube to the patient
- 15: Infeed tube for supplying the fresh dialysis fluid
- 16: Drainage tube for the used dialysis fluid
- 17: Containers of used dialysis fluid
- 18: Containers of fresh dialysis fluid
- 19: Means for positioning the auxiliary body for determining the reaching of an operative position of the selector
- 20: Engaging tooth of the auxiliary body
- 21: Elastic element of the auxiliary body
- 22: Visual marks on the auxiliary body indicating the actual operative position of the selector.
- 23: Endrun striker on the auxiliary body
- 24: Central through-cavity in the auxiliary body
- 25: Elements predisposed on the auxiliary body for engaging with corresponding elements on the valve body
- 26: Upper surface of the auxiliary body
- 27: Selector positioning device
- 28: Projection of the selector destined for insertion in the internal cavity of the valve body
- 29: T-channel internally of the selector
- 30: Window in the selector for showing the visual marks on the auxiliary body
- 31: Selector grip
- 32: Arrow showing the rotation direction of the selector

## Claims

1. A fluid control valve, comprising:
a valve body (1) having an internal cavity (2) and a plurality of fluid access ports (3) communicating with the internal cavity;
a selector (8) arranged in the internal cavity (2) and mobile among a plurality of operative positions for preventing and/or enabling fluid communication among the access ports (3);
**wherein** the valve body (1) comprises a first part (4) which at least partially defines the internal cavity (2), and a second part (5) which is connected to the first part (4) and which at least partially defines the access ports (3), the second part (5) being realised in a second material which differs from a first material of which the first part (4) is made, by at least a chemical-physical property**;**
**characterized in that:**
the second part (5) is connected unremovably to the first part (4) and is a product which is overmoulded on the first part (4); and
the at least a chemical-physical property is crystallinlty, which is higher in the first material with respect to the second material, and the first material is a crystalline or semi-crystalline plastic material.

2. The valve of claim 1, wherein the first part (4) comprises a central nucleus which is circumferentially embraced by the second part (5).

3. The valve of any one of the preceding claims, wherein the second part (5) is in contact with the first part (4).

4. The valve of any one of the preceding claims, wherein the first material is different from the second material.

5. The valve of any one of the preceding claims, wherein the second material is an amorphous plastic material.

6. The valve of any one of the preceding claims, wherein the first material comprises a polybutylene terephthalate.

7. The valve of any one of the preceding claims, wherein the second material comprises a polycarbonate.

8. The valve of any one of the preceding claims, wherein the first part (4) of the valve body has an internal surface having an axial-symmetric shape which defines the internal cavity (2), and wherein the selector (8) has an external surface which is rotatably coupled with the internal surface.

9. The valve of any one of the preceding claims, wherein the first part (4) of the valve body defines a plurality of conduits, each of which conduits connects a respective fluid access port (3) with the internal cavity.

10. A peritoneal dialysis set, comprising:
a valve (13) made as in any one of the preceding claims;
a plurality of fluid transport tubes (14, 15, 16), each of which is coupled to a respective access port (3) of the valve (13).

11. The set of claim **10**, wherein the fluid access ports (3) are at least three in number, and wherein the plurality of tubes comprises at least one tube (14) for connecting with the peritoneal cavity of a patient, an infeed tube (15) of a fresh dialysis fluid, and a drainage tube (16) of a used dialysis fluid.

## Patentansprüche

1. Fluidsteuerungsventil umfassend:
einen Ventilkörper (1) mit einer Innenausnehmung (2) und eine Vielzahl von Fluidzugangsanschlüssen (3), die mit der Innenausnehmung kommunizieren;
einen Wechselventil (8), der in der Innenausnehmung (2) angeordnet ist und zwischen einer Vielzahl von Betriebsstellungen zur Verhinderung und/oder Ermöglichung der Fluidkommunikation zwischen den Zugangsanschlüssen (3) bewegbar ist;
wobei der Ventilkörper (1) einen ersten Teil (4), der die Innenausnehmung (2) mindestens teilweise definiert, und einen zweiten Teil (5), der mit dem ersten Teil (4) verbunden ist und die Zugangsanschlüsse (3) mindestens teilweise definiert, umfasst, wobei der zweite Teil (5) aus einem zweiten Material besteht, das sich vom Material, aus dem der erste Teil (4) besteht, um mindestens eine chemisch-physikalische Eigenschaft unterscheidet;
**dadurch gekennzeichnet, daß:**
der zweite Teil (5) mit dem ersten Teil (4) fest verbunden ist und als am ersten Teil (4) übergeformtes Produkt ausgebildet ist; und
die mindestens eine chemisch-physikalische Eigenschaft Kristallinität ist, die im ersten Material als im zweiten Material höher ist, und das erste Material ein kristalliner oder halbkristalliner Kunststoff ist.

2. Ventil nach Anspruch 1, worin der erste Teil (4) einen Mittelkern umfasst, der vom zweiten Teil (5) umkreislich umgegeben ist.

3. Ventil nach irgendeinem der vorherigen Ansprüche, worin der zweite Teil (5) mit dem ersten Teil (4) in Berührung steht.

4. Ventil nach irgendeinem der vorherigen Ansprüche, worin das erste Material sich vom zweiten Material unterscheidet.

5. Ventil nach irgendeinem der vorherigen Ansprüche, worin das zweite Material ein amorpher Kunststoff ist.

6. Ventil nach irgendeinem der vorherigen Ansprüche, worin das erste Material ein Polybutylenterephthalat umfasst.

7. Ventil nach irgendeinem der vorherigen Ansprüche, worin das zweite Material ein Polykarbonat umfasst.

8. Ventil nach irgendeinem der vorherigen Ansprüche, worin der erste Teil (4) des Ventilkörpers eine Innenfläche mit einer axial symmetrischen Gestalt besitzt, die die Innenausnehmung (2) definiert, und worin der Wechselventil (8) eine Außenfläche besitzt, die mit der Innenfläche drehbar gekoppelt ist.

9. Ventil nach irgendeinem der vorherigen Ansprüche, worin der erste Teil (4) des Ventilkörpers eine Vielzahl von Leitungen definiert, wobei jede davon einen jeweiligen Fluidzugangsanschluss (3) mit der Innenausnehmung verbindet.

10. Peritonealdialyse-Set umfassend:
ein Ventil (13) nach irgendeinem der vorherigen Ansprüche;
eine Viezahl von Fluidbeförderungsrohren (14, 15, 16), wobei jedes davon mit einem jeweiligen Zugangsanschluss (3) des Ventils (13) gekoppelt ist.

11. Set nach Anspruch 10, worin die Fluidzugangsanschlüsse (3) mindestens drei sind, und worin die Vielzahl von Rohren mindestens ein Rohr (14) zur Verbindung mit der Peritonealhöhle eines Patienten, ein Zuführungsrohr (15) für ein frisches Dialysefluid und ein Ablassrohr (16) für ein gebrauchtes Dialysefluid umfasst.

## Revendications

1. Soupape de réglage de fluid comprenant:
un corps de soupape (1) ayant une cavité intérieure (2) et une pluralité de portes d'accès de fluide (3) communiquant avec la cavité intérieure;
un sélecteur (8) rangé dans la cavité intérieure (2) et mobile entre une pluralité de positions de travail pour éviter et/ou permettre la communication de fluide parmi les portes d'accès (3);
le corps de soupape (1) comprenant une première partie (4) définissant au moins partiellement la cavité intérieure (2), et une deuxième partie (5) reliée à la première partie (4) et définissant au moins partiellement les portes d'accès (3), la deuxième partie (5) étant réalisée dans un deuxième matériau différant d'un premier matériau avec lequel est réalisée la première partie (4) par au moins une propriété chimique-physique;
**caractérisée en ce que**:
la deuxième partie (5) est reliée de façon fixe à la première partie (4) et est un produit surmoulé sur la première partie (4); et
l'au moins une propriété chimique-physique est la cristallinitité, qui est supérieure dans le premier matériau par rapport au deuxième matériau, et le premier matériau est un matériau plastique cristallin ou semi-cristallin.

2. Soupape selon la revendication 1, où la première partie (4) comprend un noyau central encadré périmétralement par la deuxième partie (5).

3. Soupape selon une quelconque des revendications précédentes, où la deuxième partie (5) est en contact avec la première partie (4).

4. Soupape selon une quelconque des revendications précédentes, où le premier matériau est différent du deuxième matériau.

5. Soupape selon une quelconque des revendications précédentes, où le deuxième matériau est un matériau plastique amorphe.

6. Soupape selon une quelconque des revendications précédentes, où le premier matériau comprend un téréphtalate de polybutylène.

7. Soupape selon une quelconque des revendications précédentes, où le deuxième matériau comprend un polycarbonate.

8. Soupape selon une quelconque des revendications précédentes, où la première partie (4) du corps de soupape présente une surface intérieure ayant une forme axialement symétrique qui définit la cavité intérieure (2), et où le sélecteur (8) présente une surface extérieure couplée de façon tournante avec la surface intérieure.

9. Soupape selon une quelconque des revendications précédentes, où la première partie (4) du corps de soupape définit une pluralité de conduits, chacun reliant une porte d'accès de fluide (3) respective à la cavité intérieure.

10. Set pour dialyse péritonéale comprenant:
une soupape (13) selon une quelconque des revendications précédentes;
une pluralité de tuyaux de transport de fluide (14, 15, 16), chacun étant couplé avec une porte d'accès (3) respective de la soupape (13).

11. Set selon la revendication 10, où les portes d'accès de fluide (3) sont au moins trois, et où la pluralité de tuyaux comprend au moins un tuyau (14) apte à se relier à la cavité péritonéale d'un patient, un tuyau d'alimentation (15) pour un fluide de dialyse frais et un tuyau de drainage (16) pour un fluide de dialyse usé.
